(19)

Europäisches
Patentamt
European
Patent Office
Office européen
des brevets

(11)  **EP 3 238 112 B1**

(12)  **EUROPEAN PATENT SPECIFICATION**

(45) Date of publication and mention
of the grant of the patent:
**27.10.2021  Bulletin 2021/43**

(51) Int Cl.:
*G16B 10/00* *(2019.01)*       *G16B 30/10* *(2019.01)*

(21) Application number: **15826056.2**

(86) International application number:
**PCT/IB2015/059826**

(22) Date of filing: **21.12.2015**

(87) International publication number:
**WO 2016/103148 (30.06.2016 Gazette 2016/26)**

(54)  **METHOD AND SYSTEM FOR ASSIGNING A SPECIES TO A PLURALITY OF SEQUENCING READS**

SYSTEME, VERFAHREN UND VORRICHTUNGEN ZUR SEQUENZAUSRICHTUNG

SYSTÈMES, PROCÉDÉS ET APPAREILS D'ALIGNEMENT DE SÉQUENCES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **23.12.2014  US 201462095892 P**

(43) Date of publication of application:
**01.11.2017  Bulletin 2017/44**

(73) Proprietor: **Koninklijke Philips N.V.
5656 AG Eindhoven (NL)**

(72) Inventor: **KAMALAKARAN, Sitharthan
5656 AE Eindhoven (NL)**

(74) Representative: **Philips Intellectual Property &
Standards
High Tech Campus 5
5656 AE Eindhoven (NL)**

(56) References cited:
**WO-A1-2014/066816       US-A1- 2008 195 612
US-A1- 2011 246 084       US-B1- 8 214 153**

**Description**

BACKGROUND

Sepsis is a severe immune response that can cause leaky blood vessels, blood clots, organ failure, and death. It is estimated that over a million patients in the United States become septic, and the mortality rate is between 28-50%. (Hall MJ, Williams SN, DeFrances CJ, Golosinskiy A. Inpatient care for septicemia or sepsis: A challenge for patients and hospitals. NCHS data brief, no 62. Hyattsville, MD: National Center for Health Statistics. 2011 and Wood KA, Angus DC. Pharmacoeconomic implications of new therapies in sepsis. PharmacoEconomics. 2004; 22(14):895-906.) Sepsis is triggered by an infection in the body. Once a septic response has been triggered by an infection, a patient's condition can deteriorate rapidly. Eliminating the source of infection quickly may be critical. A first treatment step is typically broad-spectrum antibiotics. However, the infection may be caused by a fungus, virus, or a combination of pathogens, not just bacteria. Furthermore, many infections may be caused by bacteria strains that are resistant to broad-spectrum antibiotics. Identifying the pathogen allows clinicians to choose medications that are most effective against the pathogen. The sooner the pathogen is identified, the sooner the patient may receive the most effective treatment. This may improve outcomes for patients suffering from sepsis.

Aligning unknown genetic sequences to known sequences may be an accurate method of identifying a pathogen. As genetic sequencing technology becomes more widely available, it is becoming more feasible to collect samples from patients to sequence genetic information. For example, next generation sequencing techniques have exponentially decreased the cost of sequencing organisms. This genetic information may be from infection causing pathogens, patient tissue, or other sources. Sequences of samples may be compared to databases of reference sequences to attempt to identify a pathogen. To date, thousands of microbes have been sequenced for use as reference sequences, and that number is expected to grow to the hundreds of thousands in the next few years. As the number of known sequences increases, the time and computation power required to search a database of reference sequences increases. Although the cost of sequencing samples has decreased, the growing computational cost of aligning sample sequences to reference sequences may decrease the practicality of this method of pathogen identification. It may also decrease the availability of sequence alignment for other applications such as molecular biology research, food safety, and drug discovery. Patent Publication US2011/0246084 A1 discloses the identification of a source of a target nucleic acid. The sequence of the target nucleic acid is determined by sequencing and before the termination of the sequencing process, at least a portion of the target nucleic acid is compared to reference nucleotide sequences to identify matches; if sufficient identification of the source of the target nucleic acid is not possible, the sequencing process is continued and the matching and determination is repeated. It does, however, not disclose the steps of first identifying a certain sub-index from the alignment of a test set of the plurality of sequence reads to a plurality of sub-indices, the chosen sub-index being the one with the best alignment to the test set and then aligning all of the sequence reads to this one single sub-index.

SUMMARY

An example method of assigning a species to a plurality of sequence reads includes receiving the plurality of sequence reads; selecting a test set of the plurality of sequence reads, wherein the test set may include selected ones of the plurality of sequence reads; selecting a plurality of sub-indices of an index, wherein the index may point to all sequences of a set of reference sequences corresponding to a plurality of species wherein each of the sub-indices points to sequences in the set of reference sequences included in a corresponding sector of a phylogenetic tree of the set of reference sequences; aligning the test set to the plurality of sub-indices, wherein the aligning may be performed in parallel by one or more processing units; identifying, with the one or more processing units, a certain sub-index of the plurality of sub-indices with the best alignment to the test set; aligning, with the one or more processing units, the plurality of sequence reads to the certain sub-index; and assigning the species to the plurality of sequence reads based on said aligning the plurality of sequence reads to the certain sub-index.

An example system for assigning a species to a plurality of sequence reads includes a processing unit, a memory accessible to the processing unit configured to store the plurality of sequence reads, a database accessible to the processing unit configured to store a set of reference sequences corresponding to a plurality of species, an index pointing to all sequences of the set of reference sequences, and a plurality of sub-indices of the index, wherein each of the sub-indices points to sequences in the set of reference sequences included in a corresponding sector of a phylogenetic tree of the set of reference sequences, and a display coupled to the processing unit. The processing unit is configured to perform the method of claim 1 and thereby assign a species to the plurality of sequence reads to provide to the display a determination of the species.

## BRIEF DESCRIPTION OF THE DRAWINGS

FIG. 1 is a schematic illustration of a system according to an embodiment of the disclosure.
FIG. 2 is a flow chart of a method according to an embodiment of the disclosure.
FIG. 3A is a schematic illustration of a method according to an embodiment of the disclosure.
FIG. 3B is a schematic illustration of a method according to an embodiment of the disclosure.
FIG. 4. is an illustration of an example phylogenetic tree.
FIG. 5 is a flow chart of a method according to an embodiment of the disclosure.
FIG. 6 is a schematic illustration of a method according to an embodiment of the disclosure.
FIG. 7 is a schematic illustration of a method according to an embodiment of the disclosure.

## DETAILED DESCRIPTION

The following description of certain exemplary embodiments is merely exemplary in nature and is in no way intended to limit the invention or its applications or uses. In the following detailed description of embodiments of the present systems and methods, reference is made to the accompanying drawings which form a part hereof, and in which are shown by way of illustration specific embodiments in which the described systems and methods may be practiced. These embodiments are described in sufficient detail to enable those skilled in the art to practice the presently disclosed systems and methods, and it is to be understood that other embodiments may be utilized.

The following detailed description is therefore not to be taken in a limiting sense, and the scope of the present system is defined only by the appended claims. The leading digit(s) of the reference numbers in the figures herein typically correspond to the figure number, with the exception that identical components which appear in multiple figures are identified by the same reference numbers. Moreover, for the purpose of clarity, detailed descriptions of certain features will not be discussed when they would be apparent to those with skill in the art so as not to obscure the description of the present system.

Although identification of pathogens is described, this application is provided for exemplary purposes only. The methods, systems, and apparatuses described herein may be used for a wide variety of applications not limited to pathogen identification. Other applications may include, but are not limited to, genealogy, forensics, and botany.

An infection may be caused by a pathogen such as bacteria, a virus, a fungus, a parasite, or other organism. Some infections may be caused by multiple types of organisms present at the same time.

When an infection is detected, samples may be collected by medical staff from the patient. Samples may include tissue, blood, and/or bodily fluid. The samples may then be processed to isolate the pathogen causing the infection from other materials in the sample. The infection isolate may then be analyzed by a variety of methods. The analysis may determine the pathogen type, species, drug resistance, and/or other properties.

The genetic material of the infection isolate may be sequenced. Examples of sequencing methods include single-molecule real-time sequencing, pyrosequencing, and polony sequencing. Other sequencing methods may also be used. Using high-throughput sequencing (also known as next generation sequencing), the genetic material is sequenced in parallel, which may generate thousands to millions of sequence fragments. The sequence fragments generated by the sequencing method are generally referred to as "sequence reads," or simply "reads." The reads may be anywhere from a few tens to tens of thousands of base pairs long. In some sequencing methods, the reads may be the entire length of the infection isolate sequence. The reads of the infection isolate may then be analyzed to find a match to a reference sequence. The process of matching one or more reads to a known sequence is generally referred to as alignment.

Several algorithms for aligning sequences and/or reads have been developed. Certain algorithms, such as Smith-Waterman and Needleman-Wunsch algorithms, may be used to find the optimal alignment between a read and a reference sequence. Even once an optimal alignment, there may be mismatches and/or gaps between the read and reference sequence. A gap may be due to a string of mismatched bases in a row and/or a difference in length between the reference sequence and the read. A score or other measure (e.g., total number of matched nucleic acids, length of longest gap, etc.) of how well the read and reference sequence are aligned at the optimal alignment may be provided. Optimal alignment algorithms may provide the most accurate result, but the computational intensity of most of these algorithms make them difficult to implement when a large number of reads and/or reference sequences are to be aligned.

Probabilistic algorithms have been developed that may increase the speed of sequence alignment, but at the expense of not guaranteeing the optimal alignment. These algorithms may provide a measure of probability of having found the best alignment and/or the probability of having found the closest match between a read and a reference sequence from a set of reference sequences in a database.

A family of probabilistic algorithms breaks the reads and sequences into k-mers, or "words" consisting of a number (k) of base pairs. The algorithm then searches for matches in the read k-mers and the reference sequence k-mers. An example of such an algorithm is the Basic Local Alignment Search Tool (BLAST). Another family of probabilistic algorithms apply a transform to the reads and sequences such as a Borrows-Wheeler Transform (BWT). The transform may reduce the number of identical copies of a portion of a sequence, reducing alignment time. An example of this type of alignment algorithm is the Bowtie algorithm. Other probabilistic algorithm families may be used. (Li H, Homer N, "A survey of sequence alignment algorithms for next-generation sequencing," Briefings in Bioinformatics, 2(5), 473-483, 2010.)

Many probabilistic algorithms generate secondary data structures called search indices that point to elements in the primary data structure. For example, in a book, a search index provides a list of major topics and points to the pages on which a major topic is discussed. In this application, the elements in the primary data structure are sequences. These indices may be generated for the references sequences and/or sequence reads from a sample. The search indices may provide a data structure that is optimized for searching by the chosen algorithm to find matching sequences and/or sequence segments. A search index may allow the algorithm to align the reads and sequences more rapidly and/or accurately. The trade-off for this improvement in performance may be additional databases and storage space in a memory to store the search index. Examples of search index structures include, but are not limited to, hash tables, suffix/prefix trees, binning, and linear indices. An alignment algorithm may utilize one or more search indices.

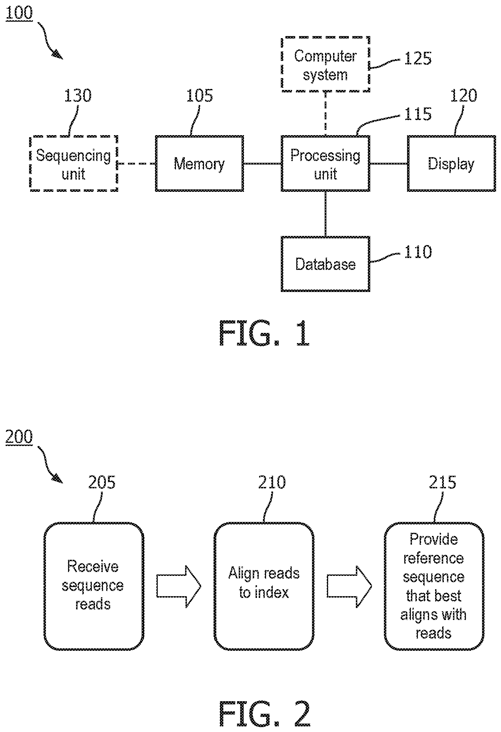

An example of a system 100 used for aligning reads to one or more reference sequences according to an embodiment of the disclosure is shown as a block diagram in Figure 1. Sequence reads from an infection isolate sample in digital form may be included in memory 105. The memory 105 may be accessible to processing unit 115. The processing unit 115 may include one or more processing units. The processing unit 115 may be configured to execute one or more alignment algorithms. The processing unit 115 may have access to a database 110 that includes one or more reference sequences and/or indices. The database 110 may include one or more databases. The processing unit 115 may provide the results of its alignment to a display 120 and/or the database 110. The display 120 may be an electronic display visible to a user. Optionally, processing unit 115 may further access a computer system 125. The computer system 125 may include additional databases, memories, and/or processing units. The computer system 125 may be a part of system 100 or remotely accessed by system 100. In some embodiments, the system 100 may also include a sequencing unit 130. The sequencing unit 130 may process an infection isolate to generate sequence reads and produce the digital form of the reads.

Figure 2 is a flow chart of an example method 200 for aligning reads to one or more reference sequences, which may be performed by a system, such as system 100 shown in Figure 1. First, sequence reads may be received at Step 205. The sequence reads may be loaded into a memory, such as memory 105. The reads may then be aligned against a search index at Step 205. The search index may point to one or more reference sequences stored in a database, such as database 110. The search index may also be stored in the database. A processing unit, such as processing unit 115, may align the reads to the search index using an alignment algorithm. The alignment algorithm may be one of the alignment algorithms described previously. After alignment, the system may provide the reference sequence or sequences that best align with the reads at Step 215. The system may provide these results to a user via a display, such as display 120, and/or another computer system, such as computer system 125. The computer system may allow the results to be accessible to other systems, for example, a hospital-wide infection tracking system or a Center for Disease Control (CDC) reporting system. Other methods of providing the reference sequence that best aligns with the reads may also be used. Other results may also be provided by the system. Other results may include, but are not limited to, percentage of match between sequences, percent probability of having found the best alignment, and errors.

The reference sequences, reads, and/or indices may be stored in different databases. The databases may be stored in different memories accessible by one or more processing units. In some cases, one or more of the databases may be divided across a plurality of memories. For example, a portion of the database of reference sequences may be stored in one memory while a second portion of the database of reference sequences may be stored in another memory. Each memory may contain a unique portion of the database or each memory may contain a portion of the database that is also stored in another memory. This may provide back-up protection in the case of a hardware failure and/or faster access for commonly used data.

Separating the data into multiple databases and/or multiple memories may allow for one or more processing units to perform alignment of reads in parallel. This may decrease computation time. For example, a search index pointing to 1,000 reference sequences may be divided into ten sub-indices each pointing to 100 reference sequences. A processing unit or processing units may access 5,000 reads in a memory and align the reads to each sub-index in parallel. This may result in an alignment in less time than if the 5,000 reads were aligned to the full search index.

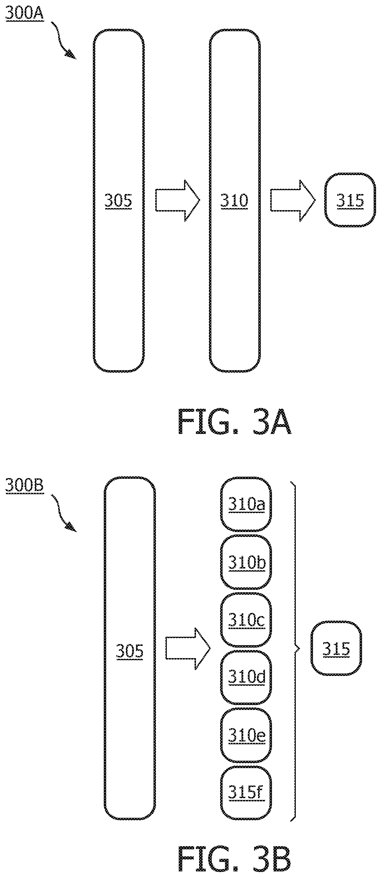

Figures 3A-B are schematic illustrations of the two methods 300A-B described above. Figure 3A illustrates a set of reads 305 aligned against a full search index 310 to provide a result 315. Figure 3B illustrates the set of reads 305 aligned against a set of sub-indices 310a-f to provide a result 315. The sub-indices 310a-f may be stored in a single database or multiple databases. The databases may be stored on one or more memories accessible by one or more processing units. The set of reads 305 may be stored in the same memory or a different memory from the sub-indices 310a-f. The sub-indices may be generated by randomly dividing the search index 310 into segments. The sub-indices 310a-f may also be generated according to a commonality between the reference sequences pointed to by a sub-index. For example, a first sub-index may point to all the reference sequences of the search index that begin with AGC, a second sub-index may point to all the reference sequences that begin with CGC, and so on. The sub-indices may be generated according to phylogenetic metrics.

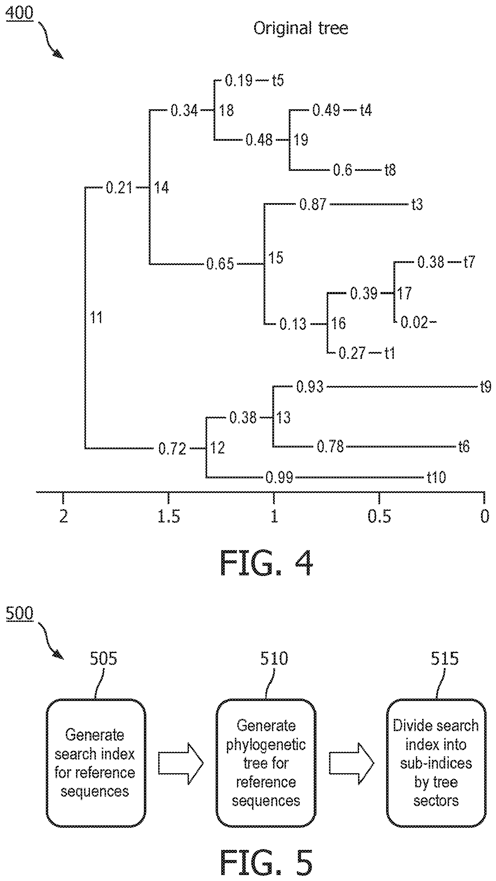

Phylogenetics is the study of evolutionary relationships between organisms. Such relationships are often represented as weighted graphs such as trees. An example of a phylogenetic tree 400 is shown in Figure 4. Different levels of granularity of data may be illustrated in a phylogenetic tree. For example, branches representing sub-species may be linked together to a single species. In another example, each branch of the tree may represent a single species. The links between branches may group several species into a larger category such as a genus. Two or more genus may be linked into a family, and so on. Additional information such as mutation rates, evolutionary distances between organisms may also be conveyed in a phylogenetic tree. For example, the length of the branches may correspond to an evolutionary distance between two organisms. Phylogenetic methods analyze all or a portion of a genetic sequence of an organism. By determining an evolutionary history of a set of reference sequences, it may be possible to organize the set of reference sequences, search index, and/or sub-indices to decrease the time required to align reads from an infection isolate to the reference sequences. The organization of the data by evolutionary history may also provide an understanding of how the pathogen of an infection isolate from a sample is related to other pathogens.

Multiple phylogenetic methods exist, including methods based on evolutionary distances, parsimonious, and maximum likelihoods. Distances based methods are where an evolutionary distance is calculated between each organism. The evolutionary distance is calculated based on the degree of similarity between genetic sequences of organisms. One such method for determining evolutionary distances is called the Jukes-Cantor (Evolution of protein molecules In Mammalian protein metabolism, Vol. III (1969), pp. 21-132 by T. H. Jukes, C. R. Cantor edited by M. N. Munro) method where the transition from any particular nucleotide in the genome to another, i.e. transitions or transversions, can occur with the same probability:

$$Q = \begin{bmatrix} -\frac{3\mu}{4} & \frac{\mu}{4} & \frac{\mu}{4} & \frac{\mu}{4} \\ \frac{\mu}{4} & -\frac{3\mu}{4} & \frac{\mu}{4} & \frac{\mu}{4} \\ \frac{\mu}{4} & \frac{\mu}{4} & -\frac{3\mu}{4} & \frac{\mu}{4} \\ \frac{\mu}{4} & \frac{\mu}{4} & \frac{\mu}{4} & -\frac{3\mu}{4} \end{bmatrix} \qquad \text{Equation 1}$$

In Equation 1, above, the instantaneous rate matrix Q represents the rates of change between a pair of nucleotides per instant of time. P - the probability transition matrix is given as

$$p(t) = e^{Qt} \qquad \text{Equation 2}$$

As a result, the evolutionary distance between any two organisms under this model is simply:

$$d_{ab} = -\frac{3}{4}\ln\left(1 - \frac{4}{3}p\right) \qquad \text{Equation 3}$$

Where p is the number of sites along the single nucleotide polymorphisms (SNPs)/DNA that differ between the sequences. The distance goes to infinity as p approaches the equilibrium value (75% of sites differ). This simple model, however, does not take into account the biological consideration that transitions (purine to purine (a-g) or pyrimidine to pyrimidine (t-c)) and transversions (purine to pyrimidine or vice-versa) occur at different rates. Another distance model, the Kimura 2-parameter model (Kimura, Motoo. "A simple method for estimating evolutionary rates of base substitutions through comparative studies of nucleotide sequences." Journal of molecular evolution 16.2 (1980): 111-120), attempts to correct for this. In this case:

$$d = -\frac{1}{2}\ln[(1 - 2p - q)(sqrt(1 - 2q))] \quad \text{Equation 4}$$

For p (proportion of transitions) and q (proportion of transversions).

Once reference sequences have been compared to determine their evolutionary distances, rates of evolution may be determined. The evolutionary distances and relationships between reference sequences may then be plotted in graphical form, such as a tree plot. Neighbor Joining (Saitou N, Nei M. "The neighbor-joining method: a new method for reconstructing phylogenetic trees." Molecular Biology and Evolution, volume 4, issue 4, pp. 406-425, July 1987) is one method of building unrooted trees. The method corrects for unequal evolutionary rates between sequences by first finding a pair of neighboring leaves i and j which have the same parent node k. That is, leaves i and j may be pathogens that evolved from a common pathogen k. Leaves i and j may then be removed from the list of leaf nodes and k is added to the current list of nodes, and node distances are recalculated. This algorithm is an example of a greedy "minimum evolution" algorithm.

Another method of building phylogenetic trees is the unweighted pair group method with arithmetic mean (UPGMA) (Sokal R., Michener C. "A statistical method for evaluating systematic relationships." University of Kansas Science Bulletin 38: 1409— 1438, 1958). The UPGMA algorithm is agglomerative and generates a rooted tree. Initially, each sequence defines a single cluster. With each iteration, clusters are combined to form larger clusters. This continues until all sequences are included in a single cluster. With each iteration, two clusters of sequences that are found to have the shortest evolutionary distance are combined into a higher-level cluster. The evolutionary distance between clusters is the average of all evolutionary distances between corresponding pairs of sequences in each of the clusters. The algorithm reiterates until all reference sequences are placed in the tree.

Single-linkage clustering is a method of building rooted trees similar to UPGMA. However, rather than using the average evolutionary distance between all corresponding pairs of sequences between clusters, the evolutionary distance between clusters is defined by the minimum distance between a sequence in a first cluster and a sequence in a second cluster. That is, the distance of a single pair of sequences defines the distance between clusters.

Complete-linkage clustering is also a method of building rooted trees similar to UPGMA and single-linkage clustering. As with single-linkage clustering, the evolutionary distance between a single pair of sequences, each included in a different cluster, defines the evolutionary distance between two clusters. However, in complete-linkage clustering, the pair of sequences that has the greatest evolutionary distance defines the evolutionary distance between the two clusters.

Unlike neighbor joining, the UPGMA algorithm and related clustering algorithms assume a constant rate of evolution. The above methods of generating phylogenetic trees are provided for example purposes only. Other methods of generating phylogenetic trees may be used without departing from the scope of the disclosure.

Figure 5 is a flow chart of a method 500 for generating a search index and sub-indices according to an embodiment of the disclosure. First, at Step 505, a search index is generated for a set of reference sequences. For example, a hash table index. Second, at Step 510, a phylogenetic tree of the set of reference sequences is generated. The phylogenetic tree may be generated by one or more of the methods described previously and/or another method. In some embodiments, Step 510 may precede Step 505. After the phylogenetic tree has been generated, the search index is divided into sub-indices at Step 515. The search index may be divided into sub-indices by sectors of the phylogenetic tree. Examples of sectors include, but are not limited to, genus, clades, branches, and phylums. Other phylogenetic metrics may also be used to constrain the sectors. For example, species within a set evolutionary distance of one another and/or species with the same mutation rate. The sub-indices may then be stored in one or more databases and/or memories. Once generated, the sub-indices may be used repeatedly for alignment of reads. However, method 500 may be repeated if the set of reference sequences is altered. For example, new reference sequences may be added or removed from the set.

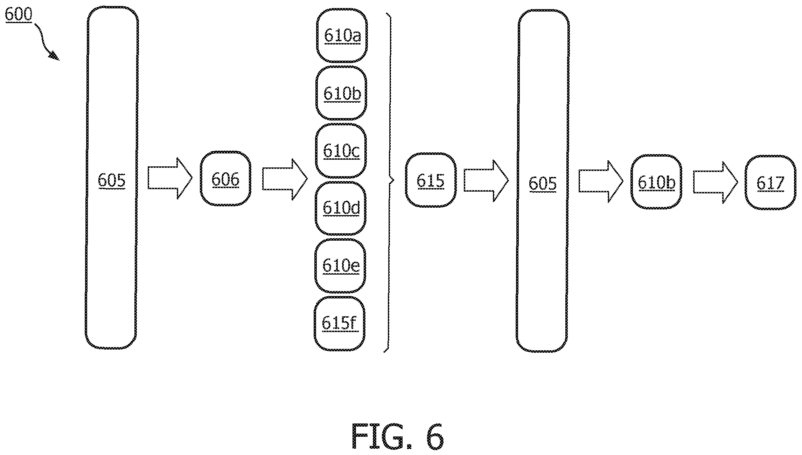

As mentioned previously, next generation sequencing methods may generate thousands to millions of reads for a single infection isolate. Even if an index has been divided into sub-indexes, aligning all of the reads to each sub-index in parallel may take a long period of time. Figure 6 is a schematic illustration of an example method 600 of dividing the reads into a subset for alignment according to an embodiment of the disclosure. A set of reads 605 may be used to generate a test set 606. The test set 606 may include a random selection of reads from the set of reads 605. The test set 606 may then be aligned with all of the sub-indices 610a-f in parallel. This may be used to produce a result of the alignment 615. The result 615 may be a reference sequence most likely to have the best alignment with the test set 606. The result 615 may be less accurate than a result, such as result 315, which utilizes all of the reads 605. Alternatively, the result 615 may indicate which sub-index 610a-f pointed to the reference sequences with the best alignment, for example, sub-index 610b. As shown in Figure 6, all of the reads 605 may then be aligned to the sub-index 610b, and the final result 617 of this alignment may then be the reference sequence most likely to have the best alignment to the reads. This reduces the number of reference sequences that the entire set of reads 605 are aligned to, which may reduce computation time.

Other permutations of subdividing the set of reads are possible. For example, the set of reads may be divided into any number of test sets, and each test set may be aligned to one or more sub-indices. The test sets may be stored in one or more memories accessible by one or more processing units. The test sets may be stored in the same memory or a different memory than the set of reads. The test sets may be stored in the same memory or different memory than the search index and/or sub-indices. The test sets, if combined, may comprise the entire set of reads. However, the test sets, if combined, may comprise only a subset of the entire set of reads. This may reduce the computation time required for aligning the test sets. The sub-index found to have the best alignment with one or more test sets may then be aligned to the complete set of reads. This step may be omitted if a less accurate result is adequate.

When the sub-indices are sectors of a phylogentic tree generated from the reference sequences pointed to by the index, the alignment may return phylogenetic information as at least a portion of the result. For example, after a set of reads generated from sequencing an infection isolate sample have been aligned to one or more sub-indices, a result may include the most likely species of the infection isolate. Other phylogenetic information may also be provided.

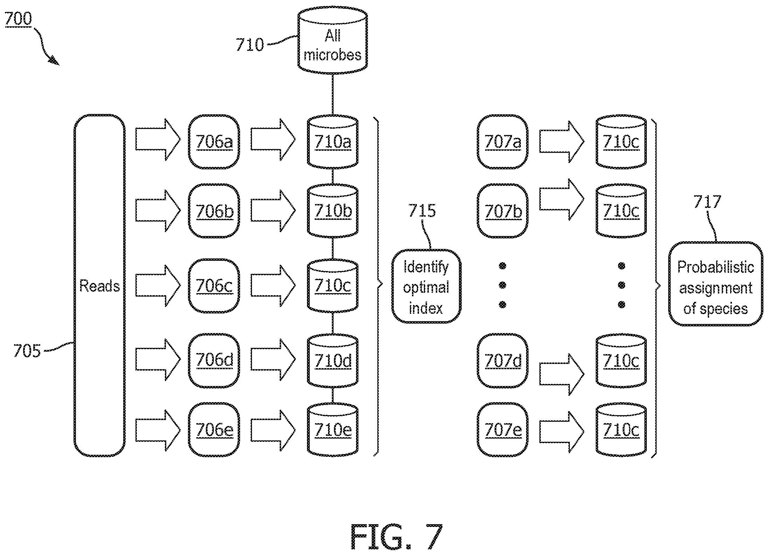

Figure 7 is a schematic illustration of assigning a species to a plurality of sequence reads 700 according to an embodiment of the disclosure. The method may be implemented by a system, such as system 100 shown in Figure 1. An infection isolate may have been obtained from a sample. The infection isolate may then have been sequenced by a sequencing technique that generated a plurality of reads. The plurality of reads may then be provided to a memory in electronic form. The memory may then contain a set of reads 705. The set of reads may then be divided into one or more test sets 706a-706e. Although six test sets are shown, any number of test sets may be generated from the set of reads 705.

A database 710 of reference sequences may also be stored in the memory or in a separate memory. The reference sequences may be the entire set of known reference sequences for all organisms or may be a subset of the entire set of known reference sequences, for example, only reference sequences from bacteria. A search index may be generated for the reference sequences in the database 710. The search index may also be stored in database 710. In some embodiments, database 710 may include multiple databases. A phylogenetic tree may be generated for the reference sequences. Based on the phylogenetic tree, one or more sub-indices 710a-e may be generated. Each sub-index 710a-e may point to reference sequences in a corresponding sector of the phylogenetic tree. For example, each sub-index 710a-e may represent a clade of the phylogentic tree of the reference sequences. Although five sub-indices are shown, any number of sub-indices may be generated from the search index. The generation of the search index and sub-indices may be performed prior to receiving a set of reads. The generation of the search index and sub-indices may only need to be performed once, and the resulting search index and sub-indices may be utilized multiple times for any number of sets of reads.

One or more processing units may access the test sets 706a-e and align each test set 706a-e to a corresponding sub-index 710a-e. The alignment of each test set with each sub-index may be performed in parallel. In some embodiments, the test sets 706a-e may only be aligned to certain ones of the sub-indices 710a-e. For example, some sub-indices may correspond to sectors of the phylogenetic tree that are known to contain no pathogenic species. These sub-indices may then be excluded from alignment when searching for an infection isolate species. The processing unit may analyze the result 715 of the alignments and identify the sub-index with the optimal alignment or the highest probability of containing the optimal alignment. In the example shown in Figure 7, sub-index 710c is the optimal sub-index.

The set of reads 705 may then be divided into one or more sub-sets 707a-e. Although five sub-sets are shown, any number of sub-sets may be generated from the set of reads 705. The sub-sets 707a-e, when combined, may include the entire set of reads 705. The sub-sets 707a-e may be identical or different from the test sets 706a-e. For example, if the combined test sets 706a-e only included a portion of the reads of the set of reads 705, the sub-sets 707a-e may be different. In another example, more or fewer sub-sets may be generated than test sets.

One or more processing units may access the sub-sets 707a-e and align each sub-set 707a-e to the optimal index 710c in parallel. In some embodiments, multiple copies of the optimal index 710c may be generated to facilitate parallel processing of the alignment. Other methods of facilitating parallel processing may also be used. The processing unit may analyze the results of the alignments of the subsets 707a-e to the optimal sub-index 710c. The processing unit may then return a result 717 of the most likely species of the infection isolate. Probabilistic methods, as described previously, may be used for the assignment of the most likely species. Other information may also be provided with the result 717. For example, a probability that the correct species has been identified, a degree of similarity between the reference sequence of the most likely species and the sequence reads of the infection isolate, and/or other likely species may be included. The result 717 may be provided to a user on an electronic display, transmitted to an external computer system, and/or stored in a memory.

The systems, methods, and apparatuses described above may improve patient outcomes by reducing the computational time of assigning a species to sequence reads from an infection isolate. When a patient is determined to have an infection, a sample may be collected from the patient. The sample may be processed to obtain an infection isolate. The infection isolate may then be sequenced by a sequencing device that generates a plurality of sequence reads. The sequence reads may be converted into electronic form and provided to a system according to an embodiment of the disclosure to compare the sequence reads to reference sequences to determine the species of the infection isolate. The system may use one or more methods of sub-dividing of the reads and/or search index described above, which may reduce the computation time required to assign a species to the infection isolate. The species assignment of the infection isolate may allow clinicians to implement the most effective treatments against the particular pathogen infecting the patient. This may reduce the time between infection and initiation of the most effective treatment. This may also reduce treating patients with ineffective or less effective treatments which may have undesirable side effects. For example, broad spectrum antibiotic treatment may be avoided if it is determined that the infection is caused by bacteria resistant to broad spectrum antibiotics.

The systems, methods, and apparatuses described above may allow for lower cost memories, databases, and/or processing units to be used for implementation. This may increase access to sequencing and alignment capabilities.

It is to be appreciated that any one of the above embodiments or processes may be combined with one or more other embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art. Accordingly, the specification and drawings are to be regarded in an illustrative manner and are not intended to limit the scope of the appended claims.

**Claims**

1.  A method of assigning a species to a plurality of sequence reads (705), the method comprising:

    receiving the plurality of sequence reads (705);
    selecting a test set (706a) of the plurality of sequence reads (705), wherein the test set (706a) includes selected ones of the plurality of sequence reads (705);
    selecting a plurality of sub-indices (710a-e) of an index, wherein the index points to all sequences of a set of reference sequences corresponding to a plurality of species, wherein each of the sub-indices (710a-e) points to sequences in the set of reference sequences included in a corresponding sector of a phylogenetic tree of the set of reference sequences;
    aligning the test set (706a) to the plurality of sub-indices (710a-e), wherein the aligning is performed in parallel by one or more processing units (115);
    identifying, with the one or more processing units (115), a certain sub-index (710c) of the plurality of sub-indices (710a-2) with the best alignment to the test set (706a);
    aligning, with the one or more processing units (115), the plurality of sequence reads (705) to the certain sub-index (710c); and
    assigning the species to the plurality of sequence reads (705) based on said aligning the plurality of sequence reads (705) to the certain sub-index.

2.  The method of claim 1, wherein selecting the test set (706a) of the plurality of sequence reads (705) comprises selecting random ones of the plurality of sequence reads (705).

3.  The method of claim 1, wherein the test set (706a) includes a plurality of test sets (706a-e), wherein each of the plurality of test sets (706a-e) is aligned to a different one of the plurality of sub-indices (710a-e).

4.  The method of claim 1, wherein the aligning, with the one or more processing units (115), the plurality of sequence reads (705) to the certain sub-index (710c) includes grouping the plurality of sequences reads (705) into a plurality of sub-sets (707a-e), wherein each sub-set (707a-e) is aligned to the certain sub-index (710c).

5.  The method of claim 1, wherein the species is a bacteria, a virus, or a fungus.

6.  A system (100) for assigning a species to a plurality of sequence reads (705), the system comprising:

    a processing unit (115);
    a memory (105) accessible to the processing unit (115) configured to store the plurality of sequence reads (705);
    a database (110) accessible to the processing unit (115) configured to store a set of reference sequences corresponding to a plurality of species, an index pointing to all sequences of the set of reference sequences, and a plurality of sub-indices (710a-e) of the index, wherein each of the sub-indices (710a-e) points to sequences in the set of reference sequences included in a corresponding sector of a phylogenetic tree of the set of reference sequences; and
    a display (120) coupled to the processing unit (115);

wherein the processing unit (115) is configured to perform the method of claim 1 and thereby assign a species to the plurality of sequence reads (705) to provide to the display (120) a determination of the species.

7. The system of claim 6, further comprising a sequencing unit (130) configured to provide the plurality of sequence reads (705) to the memory (105).

8. The system of claim 6, further comprising a computer system (125) accessible to the processing unit (115), wherein the processing unit (115) is configured to provide the determination of the species to the computer system (125).

9. The system of claim 6, wherein the processing unit (115) is further configured to provide a probability that the determination of the species is correct.

**Patentansprüche**

1. Eine Methode zum Zuordnen einer Spezies zu mehreren Sequenzwerten (705), wobei die Methode folgende Schritte umfasst:

Abrufen der Sequenzwerte (705);
Auswählen eines Testsatzes (706a) aus den Sequenzwerten (705), wobei der Testsatz (706a) ausgewählte Sequenzwerte der mehreren Sequenzwerte (705) umfasst;
Auswählen mehrerer Unterindizes (710a-e) eines Indexes, wobei der Index auf alle Sequenzen eines Referenzsequenzsatzes verweist, die mehreren Spezies entsprechen, wobei die einzelnen Unterindizes (710a-e) auf Sequenzen des Referenzsequenzsatzes verweisen, die im entsprechenden Sektor eines Kladogramm des Referenzsequenzsatzes enthalten sind;
Alignment des Testsatzes (706a) an den mehreren Unterindizes (710a-e), wobei das Alignment von mindestens einer Verarbeitungseinheit (115) parallel durchgeführt wird;
mit der mindestens einen Verarbeitungseinheit (115) Ermitteln eines bestimmten Unterindexes (710c) der mehreren Unterindizes (710a-2) mit dem besten Alignment in Bezug auf den Testsatz (706a);
Alignment der mehreren Sequenzwerte (705) mit der mindestens einen Verarbeitungseinheit (115) in Bezug auf den entsprechenden Unterindex (710c); und
Zuordnen der Spezies zu den mehreren Sequenzwerten (705) anhand des Alignments der mehreren Sequenzwerte (705) in Bezug auf den entsprechenden Unterindex.

2. Die Methode gemäß Anspruch 1, wobei das Auswählen des Testsatzes (706a) aus den mehreren Sequenzwerten (705) das Auswählen zufälliger Sequenzwerte der mehreren Sequenzwerte (705) umfasst.

3. Die Methode gemäß Anspruch 1, wobei der Testsatz (706a) mehrere Testsätze (706a-e) umfasst, wobei die einzelnen Testsätze (706a-e) auf jeweils einen anderen der mehreren Unterindizes (710a-e) ausgerichtet ist.

4. Die Methode gemäß Anspruch 1, wobei das Alignment der mehreren Sequenzwerte (705) mit der mindestens einen Verarbeitungseinheit (115) in Bezug auf den entsprechenden Unterindex (710c) das Gruppieren der Sequenzwerte (705) in mehrere Untersätze (707a-e) umfasst, wobei die einzelnen Untersätze (707a-e) am entsprechenden Unterindex (710c) ausgerichtet wird.

5. Die Methode gemäß Anspruch 1, wobei es sich bei der Spezies um ein Bakterium, einen Virus oder einen Pilz handelt.

6. Ein System (100) zum Zuordnen einer Spezies zu mehreren Sequenzwerten (705), wobei das System Folgendes umfasst:

eine Verarbeitungseinheit (115);
einen Speicher (105), auf den die Verarbeitungseinheit (115) zugreifen kann, und auf dem die mehreren Sequenzwerte (705) gespeichert werden;
eine Datenbank (110), auf die die Verarbeitungseinheit (115) zugreifen kann, und in der ein Referenzsequenzsatz, der mehreren Spezies entspricht, ein Index, der auf alle Sequenzen des Referenzsequenzsatzes verweist, und mehrere Unterindizes (710a-e) des Indexes gespeichert werden,
wobei die einzelnen Unterindizes (710a-e) auf Sequenzen des Referenzsequenzsatzes verweisen, die im entsprechenden Sektor eines Kladogramm des Referenzsequenzsatzes enthalten sind; und

eine Anzeige (120), die mit der Verarbeitungseinheit (115) verbunden ist;
wobei die Verarbeitungseinheit (115) die Methode gemäß Anspruch 1 durchführt und hierbei den mehreren Sequenzwerten (705) eine Spezies zuordnet, um auf der Anzeige (120) eine Bestimmung der Spezies bereitzustellen.

7. Das System gemäß Anspruch 6, das zudem eine Sequenziereinheit (130) umfasst, die die mehreren Sequenzwerte (705) für den Speicher (105) bereitstellt.

8. Das System gemäß Anspruch 6, das zudem ein Computersystem (125) umfasst, auf das die Verarbeitungseinheit (115) zugreifen kann, wobei die Verarbeitungseinheit (115) die Bestimmung der Spezies für das Computersystem (125) bereitstellt.

9. Das System gemäß Anspruch 6, wobei die Verarbeitungseinheit (115) zudem die Wahrscheinlichkeit angibt, zu der das Bestimmen der Spezies korrekt ist.


**Revendications**

1. Procédé d'affectation d'espèces à une pluralité de lectures de séquences (705), le procédé comprenant :

   la réception d'une pluralité de lectures de séquences (705) ;
   la sélection d'un dispositif de test (706a) de la pluralité de lectures de séquences (705), dans lequel le dispositif de test (706a) comprend des lectures de séquences sélectionnées de la pluralité de lectures de séquences (705) ;
   la sélection d'une pluralité de sous-index (710a-e) d'un index, dans lequel les points d'index de toutes les séquences d'un ensemble de séquences de référence correspondent à une pluralité d'espèces,
   dans lequel chacun des sous-index (710-a-e) indique les séquences dans l'ensemble de séquences de référence comprises dans un secteur correspondant d'un arbre phylogénétique de l'ensemble de séquences de référence ;
   l'alignement du dispositif de test (706a) à la pluralité de sous-index (710-e), dans lequel l'alignement est réalisé en parallèle par une ou plusieurs unités de traitement (115);
   l'identification, avec une ou plusieurs des unités de traitement (115), d'un certain sous-index (710c) de la pluralité des sous-index (710-a-2) avec le meilleur alignement au dispositif de test (706a) ;
   l'alignement, avec une ou plusieurs unités de traitement (115), de la pluralité des lectures de séquences (705) au certain sous-index (710c) ; et
   l'affectation d'espèces à la pluralité de lectures de séquences (705) sur la base dudit alignement de la pluralité des lectures de séquences (705) au certain sous-index.

2. Procédé selon la revendication 1, dans lequel la sélection du dispositif de test (706a) de la pluralité des lectures de séquences (705) comprend la sélection de lectures de séquences aléatoires de la pluralité des lectures de séquences (705).

3. Procédé selon la revendication 1, dans lequel le dispositif de test (706a) comprend une pluralité de dispositifs de test (706a-e), dans lequel chaque pluralité de dispositifs de test (706a-e) est alignée sur un sous-index différent de la pluralité des sous-index (710a-e).

4. Procédé selon la revendication 1, dans lequel l'alignement, avec une ou plusieurs unités de traitement (115), de la pluralité des lectures de séquences (705) à certains sous-index (710c) comprend le groupement de la pluralité des lectures de séquences (705) en une pluralité de sous-ensembles (707a-e), dans lequel chaque sous-ensemble (707a-e) est aligné au certain sous-index (710c).

5. Procédé selon la revendication 1, dans lequel l'espèce est une bactérie, un virus, ou un champignon.

6. Système (100) pour attribuer une espèce à une pluralité de lectures de séquences (705), le système comprenant :

   une unité de traitement (115);
   une mémoire (105) accessible à l'unité de traitement (115) configurée pour stocker la pluralité de lectures de séquences (705) ;
   une base de données (110) accessible à l'unité de traitement (115) configurée pour stocker un ensemble de séquences de référence correspondant à une pluralité d'espèces, un index indiquant toutes les séquences de l'ensemble de séquences de référence, et une pluralité de sous-index (710a-e) de l'index, dans laquelle chacun des sous-index (710a-e) indique les séquences dans l'ensemble des séquences de référence comprises dans un secteur correspondant d'un arbre phylogénétique de l'ensemble de séquences de référence ; et

un écran (120) couplé à l'unité de traitement (115); dans lequel l'unité de traitement (115) est configurée pour réaliser le procédé de la revendication 1 et ainsi attribuer une espèce à la pluralité des lectures de séquences (705) pour fournir à l'écran (120) une détermination des espèces.

7. Système selon la revendication 6, comprenant en outre une unité de séquençage (130) configurée pour fournir la pluralité des lectures de séquences (705) à la mémoire (105).

8. Système selon la revendication 6, comprenant en outre un système informatique (125) accessible à l'unité de traitement (115), dans lequel l'unité de traitement (115) est configurée pour fournir la détermination des espèces au système informatique (125).

9. Système selon la revendication 6, dans lequel l'unité de traitement (115) est en outre configurée pour fournir une probabilité selon laquelle la détermination des espèces est correcte.

100

130    105                    Computer
                             system          125

Sequencing        Memory      Processing      Display
unit          115   120       unit

                              Database        110

# FIG. 1

200   205        210        215

Receive        Align reads      Provide
sequence       to index         reference
reads                           sequence
                                that best
                                aligns with
                                reads

# FIG. 2

300A

305 ⇒ 310 ⇒ 315

FIG. 3A

300B

305 ⇒ 310a
310b
310c
310d
310e
315f

315

FIG. 3B

<u>400</u>

Original tree

0.19 — t5
0.34 — 18
0.48 — 19
0.49 — t4
0.6 — t8
0.21 — 14
0.87 — t3
0.65 — 15
0.38 — t7
0.39 — 17
0.13 — 16
0.02 —
0.27 — t1
11
0.93 — t9
0.38 — 13
0.72 — 12
0.78 — t6
0.99 — t10

2          1.5          1          0.5          0

## FIG. 4

<u>500</u>

505

Generate
search index
for reference
sequences

510

Generate
phylogenetic
tree for
reference
sequences

515

Divide search
index into
sub-indices
by tree
sectors

## FIG. 5

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110246084 A1

**Non-patent literature cited in the description**

- **HALL MJ ; WILLIAMS SN ; DEFRANCES CJ ; GO-LOSINSKIY A.** Inpatient care for septicemia or sepsis: A challenge for patients and hospitals. NCHS data brief, no 62. National Center for Health Statistics, 2011
- **WOOD KA ; ANGUS DC.** Pharmacoeconomic implications of new therapies in sepsis. *PharmacoEconomics,* 2004, vol. 22 (14), 895-906
- **LI H, HOMER N.** A survey of sequence alignment algorithms for next-generation sequencing. *Briefings in Bioinformatics,* 2010, vol. 2 (5), 473-483
- **H. JUKES ; C. R. CANTOR.** Evolution of protein molecules In Mammalian protein metabolism. 1969, vol. III, 21-132
- **KIMURA, MOTOO.** A simple method for estimating evolutionary rates of base substitutions through comparative studies of nucleotide sequences. *Journal of molecular evolution,* 1980, vol. 16.2, 111-120
- **SAITOU N ; NEI M.** The neighbor-joining method: a new method for reconstructing phylogenetic trees. *Molecular Biology and Evolution,* July 1987, vol. 4 (4), 406-425
- **SOKAL R. ; MICHENER C.** A statistical method for evaluating systematic relationships. University of Kansas Science Bulletin, 1958, vol. 38, 1409-1438